(19) Europäisches Patentamt
European Patent Office
Office européen des brevets

(11) EP 4 789 534 A1

(12) EUROPEAN PATENT APPLICATION
published in accordance with Art. 153(4) EPC

(43) Date of publication:
**12.08.2026 Bulletin 2026/33**

(21) Application number: **24874773.5**

(22) Date of filing: **04.10.2024**

(51) International Patent Classification (IPC):
***A01H 1/06*** (2006.01) ***A01G 7/00*** (2006.01)
***A01G 22/22*** (2018.01) ***A01H 3/00*** (2006.01)
***A01H 5/04*** (2018.01) ***A01H 5/06*** (2018.01)
***A01H 5/10*** (2018.01) ***A01H 5/12*** (2018.01)
***A01H 6/14*** (2018.01) ***A01H 6/20*** (2018.01)
***A01H 6/46*** (2018.01) ***A01H 6/54*** (2018.01)
***C07K 14/415*** (2006.01) ***C12N 5/04*** (2006.01)
***C12N 15/29*** (2006.01) ***C12P 1/00*** (2006.01)
***C12P 21/02*** (2006.01)

(52) Cooperative Patent Classification (CPC):
**A01G 7/00; A01G 22/22; A01H 1/06; A01H 3/00; A01H 5/04; A01H 5/06; A01H 5/10; A01H 5/12; A01H 6/14; A01H 6/20; A01H 6/46; A01H 6/54; C07K 14/415; C12N 5/04; C12P 1/00;** (Cont.)

(86) International application number:
**PCT/JP2024/035680**

(87) International publication number:
**WO 2025/075174 (10.04.2025 Gazette 2025/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.10.2023 JP 2023173016**

(71) Applicant: **Kyushu University, National University Corporation**
**Nishi-ku**
**Fukuoka-shi**
**Fukuoka 819-0395 (JP)**

(72) Inventors:
- **ISHIBASHI, Yushi**
  **Fukuoka 8190395 (JP)**
- **SURIYASAK, Chetphilin**
  **Fukuoka 8190395 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) METHOD FOR PRODUCING PLANT HAVING CONTROLLED STOMATAL DENSITY AND USE THEREOF

(57) The object is to clarify the relationship between heat stress during the grain filling stage and the control of stomatal density, and thereby produce useful plants using a novel method distinct from conventional genetic engineering techniques. There is provided a method for producing a progeny plant with controlled stomatal density, comprising the step of controlling the temperature during the grain filling stage of a plant. The control of stomatal density is preferably achieved by controlling methylation of a promoter region of stomatal development related genes.



Processed by Luminess, 75001 PARIS (FR)

(Cont. next page)

[Fig. 1]

Abaxial surface
Adaxial surface
Abaxial and adaxial surfaces
Stomatal density (stomata/mm²)
CS
HDS
**
**
***
100 µm
CS
HDS

(52) Cooperative Patent Classification (CPC): (Cont.)
**C12P 21/02**

## Description

### Technical Field

The present invention relates to a method for producing a plant with controlled stomatal density and use thereof. The present invention is useful in the fields of plant improvement, crop production, industrial production of crops, and so forth.

### Background Art

Plant stomata comprise a structure including a pair of guard cells and cells surrounding them, and the size of the pore formed between the guard cells is regulated by changes in the shape of the guard cells. Through open stomata, carbon dioxide necessary for photosynthesis is taken in, oxygen produced by photosynthesis is released, and water vapor is also released into the air through transpiration. Transpiration also plays a role in lowering leaf temperature elevated by strong sunlight.

Regarding increasing the number of stomata in plants, Patent document 1 discloses A method of producing plants with an increased number of stomata relative to control like plants comprising the steps of: (i) inhibiting in plant material the production of fatty acids which stimulate the synthesis of the 14-3-3 class of transcription factors, or otherwise preventing the fatty acids from stimulating the synthesis of the said factors; (ii) selecting the thus inhibited material; and (iii) regenerating the thus selected material into plants and selecting from the population of regenerants those plants having an increased number of stomata relative to control like plants.. The sequences of SEQ ID NOS: 1 and 2 pertaining to this method relate to the fatty acid elongase gene called FAE-1. Patent document 2 discloses a stoma-increasing agent comprising a compound that increases the number and/or density of stomata in plants. The compound contained in this stoma-increasing agent is a polypeptide consisting of an amino acid sequence selected from the group consisting of: (I) the amino acid sequence of SEQ ID NO: 6; (II) an amino acid sequence derived from the amino acid sequence of SEQ ID NO: 6 by substitution, deletion, addition or insertion of one or several amino acid residues and constituting a polypeptide having at least a property of positively regulating stomatal formation in plants; (III) an amino acid sequence having a sequence identity of 95% or higher to the amino acid sequence of SEQ ID NO: 6 as calculated by alignment based on the BLAST algorithm under the conditions of Cost to open gap 11, Cost to extend gap 1, expect value 10, and Word size 3, where the encoded polypeptide has at least a property of positively regulating stomatal formation in plants, and (IV) an amino acid sequence encoded by a nucleic acid that hybridizes with the complementary strand nucleic acid of the nucleic acid encoding the amino acid sequence of SEQ ID NO: 6 under stringent conditions, where the encoded polypeptide has at least a property of positively regulating stomatal formation in plants. The sequence of SEQ ID NO: 6 is the sequence of the EPFL9 gene (At4g12970) belonging to the EPF family (stomagen gene), of which functions are unknown.

Meanwhile, environmental stresses such as high temperature, drought, and salinity can cause losses in crop production, and consequently, various studies have been conducted on the effects of environmental stresses on crops and methods for enhancing crop resistance to such stresses. For example, Patent document 3 proposes a method for enhancing stress resistance in the next generation of plants, characterized by including a step of applying a stress treatment to a plant of the current generation during at least the vegetative growth stage of the plant. This reference describes in the section of Examples that when seeds of the rice varieties Hitomebore and Sasanishiki were obtained with applying salt stress or low light stress during the vegetative growth stage, the next generations of rice cultivated from these seeds exhibited enhanced cold tolerance during the booting stage.

It has recently been revealed that plants can remember past environmental events and utilize this memory to respond when similar events recur. The epigenetic mechanisms employed by plants under various environmental stresses are being elucidated, and it is known that these mechanisms play crucial roles in regulating gene expression via small RNAs, histone modifications, DNA methylation, and so forth (Non-patent document 1). Research on the mechanism of seed germination delay in rice (*Oryza sativa* L.) due to high temperatures during the grain filling stage has been conducted, and it has been reported that high temperatures during ripening cause DNA methylation in the promoters of abscisic acid (ABA) catabolism-related genes and the $\alpha$-amylase gene (Non-patent document 2). Further, regarding wheat, the effect of heat priming applied to the first generation on the next generation's tolerance to post-flowering high temperature stress has been reported (Non-patent document 3). This report describes that the progeny of heat-primed plants (PH) exhibited higher grain yield, leaf photosynthesis, antioxidant enzyme activity, and less cell membrane damage under high temperature stress compared with the progeny of the plants not applied the heat priming (NH), and the gene encoding lysine-specific histone demethylase 1 (LSD1), which is associated with epigenetic modifications, showed increased expression in PH compared with NH, and concluded that applying heat priming to the first generation induces transgenerational heat tolerance and can be an effective means to address high temperature stress in wheat production.

### Prior Art References

Patent documents

Patent document 1: International Publication WO99/54471 (Japanese Patent Publication (Kohyo) No. 2002-512035)
Patent document 2: International Publication WO2011/071050 (Japanese Patent No. 5825574)
Patent document 3: Japanese Patent Publication (Kokai) No. 2015-181370
Patent document 4: International Publication WO2023/191038

Non-patent documents

Non-patent document 1: Kinoshita, T. et al., Epigenetic Memory for Stress Response and Adaptation in Plants. Plant Cell Physiol., 55(11): 1859-1863 (2014)
Non-patent document 2: Suriyasak, C. et al., Mechanism of delayed seed germination caused by high temperature during grain filling in rice (Oryza sativa L.). Scientific Reports, 10: 17378 (2020)
Non-patent document 3: Heat Priming Induces Trans-generational Tolerance to High Temperature Stress in Wheat. Front. Plant Sci., 7: Article501 (2016)

Summary of the Invention

Problem to be solved by the invention

The inventors of the present invention previously discovered that if seeds are obtained from rice plants cultivated under high temperature stress during the grain filling stage, the plants cultivated from the obtained seeds exhibit delayed germination, increased biomass, accelerated flowering, increased grain yield, and high temperature grain filling tolerance. Furthermore, they also found that such environmental stress is imprinted in the seeds, enabling the production of seeds for agricultural crop production with enhanced environmental stress tolerance (Patent document 4).
However, the effect of the environment during the grain filling stage on the stomatal density of the next generation remains unclear.

Means for solving the problem

The inventors of the present invention found that if seeds obtained from plants grown under high temperature stress during the grain filling stage are cultivated, stomatal density in the next generation of plants is controlled, and thus accomplished the present invention.
The present invention provides the following.

[1] A method for producing a next generation plant with controlled stomatal density, comprising the step of cultivating a plant under environmental stress.
[2] The production method according to [1], wherein the step of cultivating a plant under environmental stress comprises the step of controlling the temperature during the grain filling stage of the plant.
[3] The production method according to [1] or [2], wherein the control of stomatal density is achieved by controlling methylation of a promoter region of stomatal development related genes.
[4] The production method according to any one of [1] to [3], wherein the control of stomatal density is achieved by controlling methylation of a promoter region of a gene encoding any one of the following proteins:

(A) a protein consisting of the amino acid sequence of SEQ ID NO: 1;
(B) a protein consisting of an amino acid sequence derived from the amino acid sequence of SEQ ID NO: 1 by substitution, deletion, addition, or insertion of one or several amino acids, provided that the portion corresponding to the portion consisting of the amino acids at positions 236 to 330 is identical, and having a property of controlling stomatal formation in plants; and
(C) a protein consisting of an amino acid sequence having a sequence identity of 60% or higher to the amino acid sequence of SEQ ID NO: 1, provided that the portion corresponding to the portion consisting of the amino acids at positions 236 to 330 is identical, and having a property of controlling stomatal formation in plants.

[5] The production method according to [4], wherein expression of the gene is reduced by controlling methylation of the

promoter region of the gene, thereby producing a next generation plant with increased stomatal density.

[6] A plant with increased stomatal density obtainable by the method according to any one of [1] to [5], a seed, a seedling, or a processed product thereof.

[7] A method for fixing atmospheric carbon dioxide using the plant according to [6].

[8] A method for cultivating the plant according to [6], comprising the step of applying a material by foliar spray.

[9] A method for controlling stomatal density of a next generation plant, comprising the step of cultivating a plant under environmental stress.

[10] The method according to [9], wherein the step of cultivating a plant under environmental stress comprises the step of controlling the temperature during the grain filling stage of the plant.

Effect of the Invention]

Next-generation plants with controlled stomatal density can be produced by using existing superior varieties without genetic manipulation or breeding through hybridization and fixation.

Plants with increased stomatal density can absorb more materials from their leaf surfaces.

Plants with increased stomatal density can fix more carbon dioxide from the atmosphere.

Brief Description of the Drawings

[Fig. 1] Increase in stomatal density during rice growth stages (field trial). CS, Control (22 to 32°C) developed seeds; HDS, Heat (28 to 38°C) developed seeds. n=4. Significant differences at P<0.01** and P<0.001*** in Student's t-test are indicated.

[Fig. 2] Increases in stomatal density (n=5) and stomatal conductance (n=3) (pot test). CS, Control (22 to 32°C) developed seeds: HDS, Heat (28 to 38°C) developed seeds. n=3. Significant differences at P<0.01** and P<0.001*** in Student's t-test are indicated.

[Fig. 3] Increase in biomass under high $CO_2$ conditions. CS, Control (22 to 32°C) developed seeds; HDS, Heat (28 to 38°C) developed seeds. n=13. Significant differences at P<0.01** and P<0.001*** in Student's t-test are indicated.

[Fig. 4] Identification of the causative gene involved in increase in stomatal density (OsYODA1). CS, Control (22 to 32°C) developed seeds; HDS, Heat (28 to 38°C) developed seeds. n=3. Significant differences at P<0.05* and P<0.01** in Student's t-test are indicated

[Fig. 5] Methylation level in the OsYODA1 promoter region that regulates gene expression of OsYODA1. CS, Control (22 to 32°C) developed seeds; HDS, Heat (28 to 38°C) developed seeds. n=4. Significant differences at P<0.05*, P<0.01**, and P<0.001*** in Student's t-test are indicated.

[Fig. 6] Increase in stomatal density in lettuce (*Lactuca sativa*). n=4. Significant differences at P<0.05* in Student's t-test are indicated.

[Fig. 7] Changes in photosynthetic rate, stomatal aperture, and transpiration rate due to increased stomatal density in lettuce (*Lactuca sativa*).

[Fig. 8] YODA1 gene expression and methylation level in seeds of lettuce (*Lactuca sativa*). n=3. Significant differences at P<0.05* and P<0.01** in Student's t-test are indicated.

[Fig. 9] Stomatal density (n=10), biomass reduction (n=25), and AtYODA1 gene expression (n=3) in *Arabidopsis thaliana*. For biomass reduction (bottom left, bottom center, and bottom right). Significant differences at P<0.01** and P<0.001*** in Student's t-test are indicated. For AtYODA1 gene expression (top right), n=3. Significant differences at P<0.05* and P<0.01** in Student's t-test are indicated.

[Fig. 10] Increase in stomata and high temperature tolerance in soybean (*Glycine max* (L.) Merr). n=4. Significant difference at P<0.01** in Student's t-test is indicated.

[Fig. 11] Increase in photosynthetic rate in soybean (*Glycine max* (L.) Merr). n=3. Significant differences at P<0.05* and P<0.01** in Student's t-test are indicated.

[Fig. 12] Reduction in stomatal density and high temperature tolerance in barley (*Hordeum vulgare* L.). n=4. Significant difference at P<0.05* in Student's t-test is indicated.

[Fig. 13] Reduction in stomatal density in wheat (*Triticum aestivum* L.). n=4. Significant difference at P<0.01** in Student's t-test is indicated.

[Fig. 14] Increase in stomatal density in corn (*Zea mays*). n=3. Significant differences at P<0.05* and P<0.01** in Student's t-test are indicated.

[Fig. 15] Increase in stomatal density in strawberry (*F.* x *ananassa*). n=3. Significant differences at P<0.05* and P<0.01** in Student's t-test are indicated.

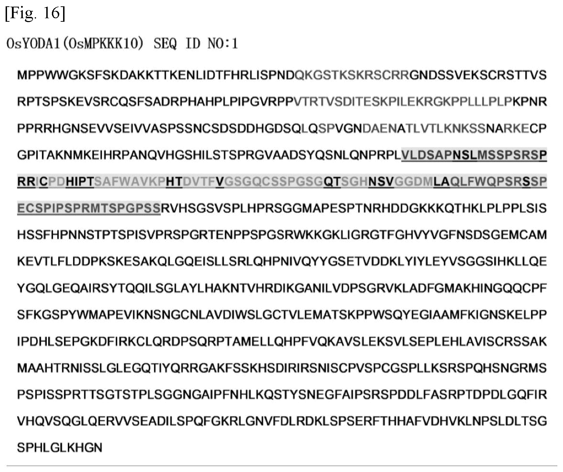

[Fig. 16] Amino acid sequence of OsYODA1 (OsMPKKK10) (SEQ ID NO: 1). The underlined region is the conserved

N-terminus regulatory domain.

Modes for Carrying out the Invention

[Control of stomatal density]

The present invention relates to a method for controlling stomatal density of next generation plants, comprising the step of exposing a plant to environmental stress. By controlling stomatal density of next generation plants, next generation plants with controlled stomatal density can be produced. Therefore, the present invention provides, as one aspect thereof, a method for producing a next generation plant with controlled stomatal density, comprising the step of exposing a plant to environmental stress.

In a preferred embodiment, the step of exposing a plant to environmental stress includes the step of controlling the temperature during the grain filling stage of the plant. By controlling the stomatal density of the next generation plants, next-generation plants with controlled stomatal density can be produced. Therefore, one aspect of the present invention is a method for producing a next generation plant with controlled stomatal density, comprising the step of controlling the temperature during the grain filling stage of a plant.

Stomatal density is expressed by the number of stomata per unit area (number/$mm^2$). Stomata are found on various plant parts such as leaves, sepals, and petals. However, unless especially stated, "stomatal density" in the context of the present invention refers to leaf stomatal density. Stomata are typically observed on both the abaxial surface (underside) and the adaxial surface (upper side) of leaves. The method of the present invention can control the stomatal density on both surfaces. Plant stomatal density can be determined by methods well known to those skilled in the art, such as the SUMP method. The control includes both upward control (increasing stomatal density) and downward control (decreasing stomatal density).

In this aspect of the present invention, the control of plant stomatal density is achieved by controlling the temperature during the grain filling stage of the plant of parent generation. More precisely, by controlling methylation of the promoter region of a gene involved in stomatal density, a plant of the next generation with controlled stomatal density can be produced. Methylation in the promoter region of genes is known to be associated with their transcriptional control (Zhang et al. 2006).

The gene involved in stomatal density is any one selected from FAMA, MUTE, SPCH1, SPCH2, YODA1, YODA2, ICE1, ICE2, SHR1, SHR2, SCR1, SCR2, SDD1, RSD1, EPF1, EPFL9, FLP, and PAN2. In a preferred embodiment, a plant of the next generation with controlled stomatal density is produced by regulating the transcription or expression of the YODA1 gene among the genes involved in stomatal density. YODA1 is known to suppress stomatal formation by inhibiting expression of SPCH (Samakovli et al. 2020, Molecular Plants).

The YODA1 gene can be a gene encoding any one of the following proteins:

(A) a protein consisting of the amino acid sequence of SEQ ID NO: 1;
(B) a protein consisting of an amino acid sequence derived from the amino acid sequence of SEQ ID NO: 1 by substitution, deletion, addition, or insertion of one or several amino acids, provided that the portion corresponding to the portion consisting of the amino acids at positions 236 to 330 of the amino acid sequence of SEQ ID NO: 1 is identical to that of the amino acid sequence of SEQ ID NO: 1, and having a property of controlling stomatal formation in plants; and
(C) a protein consisting of an amino acid sequence having a sequence identity of 60% or higher to the amino acid sequence of SEQ ID NO: 1, provided that the portion corresponding to the portion consisting of the amino acids at positions 236 to 330 of the amino acid sequence of SEQ ID NO: 1 is identical to the portion of the amino acid sequence of SEQ ID NO: 1, and having a property of controlling stomatal formation in plants.

The YODA1 gene may be a gene encoding any one of the following proteins:

(A) a protein consisting of the amino acid sequence of SEQ ID NO: 1;
(B) a protein consisting of an amino acid sequence derived from the amino acid sequence of SEQ ID NO: 1 by substitution, deletion, addition, or insertion of one or several amino acids, provided that the portion corresponding to the portion consisting of the amino acids at positions 236 to 330 is identical, and having a property of controlling stomatal formation in plants; and
(C) a protein consisting of an amino acid sequence having a sequence identity of 60% or higher to the amino acid sequence of SEQ ID NO: 1, provided that the portion corresponding to the portion consisting of the amino acids at positions 236 to 330 is identical, and having a property of controlling stomatal formation in plants.

The amino acid sequence of OsYODA1 (OsMPKKK10) derived from rice (*Oryza sativa* L. cv. Nipponbare) is

shown in Sequence Listing as SEQ ID NO: 1.

In the present invention, the term sequence identity for amino acid sequence or nucleotide sequence (sometimes referred to as base sequence) means the percentage of numbers of matching amino acids or nucleotides shared by two sequences aligned in the optimal manner, unless especially noted. That is, the identity can be calculated in accordance with the following equation: Sequence identity = (Number of matched positions/Total number of positions) x 100. This calculation can be carried out by using commercially available algorithms. Such algorithms are incorporated into the NBLAST and XBLAST programs described in Altschul et al., J. Mol. Biol. 215 (1990) 403-410. The calculation of nucleotide sequence identity can be performed by using programs well known to those skilled in the art (e.g., BLASTN, BLASTP, BLASTX, and ClustalW). When using these programs, parameters can be appropriately set by those skilled in the art, or the default parameters of each program may be used. The specific techniques for these analysis methods are also well known to those skilled in the art.

For the present invention, the term high sequence identity used for sequences means, in all cases, sequence identity of 60% or higher, 64% or higher, 65% or higher, 69% or higher, 70% or higher, 72% or higher, 75% or higher, 76% or higher, 77% or higher, 78% or higher, or 79% or higher, preferably 80% or higher, 81% or higher, 83% or higher, or 84% or higher, more preferably 85% or higher, 86% or higher, or 89% or higher, further preferably 90% or higher, further preferably 95% or higher, further preferably 97.5% or higher, further preferably 99% or higher, unless especially noted.

For the present invention, the number of amino acids or nucleotides (sometimes referred to as bases) mentioned in the expression that substitution, deletion, addition, or insertion of one or several amino acids or nucleotides used for sequences is, in any case, 1 to 400, 1 to 350, 1 to 300, 1 to 250, 1 to 200, 1 to 150, 1 to 100, 1 to 90, 1 to 80, 1 to 70, 1 to 60, 1 to 50, 1 to 40, 1 to 30, 1 to 20, 1 to 18, 1 to 16, 1 to 14, 1 to 12, 1 to 10, or 1 to 9, unless especially noted.

The sequence identities between rice-derived OsYODA1 and homologues thereof derived from other plants (analyzed in https://phytozome-next.jgi.doe.gov) are as shown in the following tables.

[Table 1-1]

**Protein similarities in other species: Whole sequence similarity**

| Plant species | Scientific name | Transcript name | Protein similarity (%) |
|---|---|---|---|
| Rice | *O. sativa* | LOC_Os04g47240.1 | 100% |
| Common wheat | *T. aestivum* | Traes_2AL_C93FB1762.2<br>Traes_2DL_8AB4895C2.2 | 92.3%<br>91.5% |
| Brachypodium distachyon | *B. distachyon* | Bradi5g18180.1 | 95.2% |
| Soybean | *G. max* | Glyma.09G135100.1 | 72.8% |
| Poplus | *P. deltoides* | Podel.15G149000.1 | 73.1% |
| Barley | *H. vulgare* | HORVU2Hr1G096350.1 | 91.7% |
| Yam | *D. alata* | Dioal.2032s0002.1 | 78.7% |
| Foxtail millet | *S. italica* | Seita.7G196800.1 | 90.8% |
| Pineapple | *A. comosus* | Aco012013.1 | 79.4% |
| Sorghum | *S. bicolor* | Sobic.006G176800.1 | 90.3% |
| Maize | *Z. mays* | ZmPHB47.02G070300.1 | 89.5% |
| Cowpea | *V. unguiculata* | Vigun07g223800.1 | 73.0% |
| Domesticated Sunflower | *H. annuus* | HanXRQChr10g0304211 | 72.3% |
| Common Grape | *V. vinifera* | VIT_202s0025g03370.1 | 74.6% |
| Citrus | *C. clementina* | Ciclev10004272m | 74.0% |
| Tomato | *S. lycopersicum* | Solyc03g025360.2.1 | 72.6% |
| Cassava | *M. esculenta* | Manes.06G150300.1 | 74.4% |
| Thale cress | *A. thaliana* | AT1G63700.1 | 72.2% |
| Lettuce | *L. sativa* | Lsat_1_v5_gn_8_7320.1 | 72.0% |
| Strawberry | *F.x ananassa* | maker-Fvb7-4-augustus-gene-21.56-mRNA-1 | 72.7% |

[Table 1-2]

**Protein similarities in other species: Conserved N-terminal regulatory domain**

| Order | Plant species | Scientific name | Transcript name | Protein similarity (%) |
|---|---|---|---|---|
| **Poales -** | **Rice** | ***O. sativa*** | **LOC_Os04g47240.1** | **100%** |
| Brassicales | Thale cress | *A. thaliana* | AT1G63700 | 64% |
| | Turnip mustard | *B. rapa* | Brara.I01384.1.p | 76% |
| | Papaya | *C. papaya* | evm.model.supercontig_4129.1 | 73% |
| Malvales | Cacao | *T. cacao* | Thecc1EG016276t1 | 77% |
| Sapindales | Orange | *C. sinesis* | orange1.1g013380m | 67% |
| Myrtales | Eucalyptus | *E. grandis* | Eucgr.A02762.1 | 60% |
| Malpighiales | Poplar | *P. trichocarpa* | Potri.012G143900.10.p | 74% |
| Fabales | Soybean | *G. max* | Glyma.16G181000.1 | 73% |
| | Cowpea | *V. unguiculata* | Vigun07g223800.1 | 74% |
| Cucurbitales | Cucumber | *C. sativus* | Cucsa.107950.1 | 64% |
| Rosales | Strawberry | *F.x ananassa* | maker-Fvb7-4-augustus-gene-21.56-mRNA | 77% |
| Vitales | Common grape | *V. vinifera* | VIT_216s0050g00770.7 | 79% |
| Asterales | Lettuce | *L. sativa* | Lsat_1_v5_gn_8_7320.1 | 68% |
| | Sunflower | *H. annuus* | HanXRQChr06g0165921 | 72% |
| Lamiales | Olive | *O. europaea* | Oeu064568.1 | 71% |
| Sonanales | Tomato | *S. lycopersicum* | Solyc08g081210.2.1 | 76% |
| Caryophylalles | Amanranth | *A. hypochondriacus* | AH012136-RA\|AH012136-RA | 72% |
| | Sugar beet | *B. vulgaris* | EL10Ac7g17909.1 | 69% |
| | Spinach | *S. oleracea* | Spov3_chr3.04659 | 74% |
| Ranaculales | Colorado blue columbine | *A. coerulea* | Gohir.D09G043100.3 | 74% |
| Poales | Maize | *Z. mays* | Zm00001d051404_P023 | 92% |
| | Wheat | *T. aestivum* | Traes_2BL_85B8826FB.7 | 97% |
| | Barley | *H. vulgare* | HORVU2Hr1G096350 | 97% |
| Zingiberales | Banana | *M. acuminata* | GSMUA_Achr4P25300_001 | 87% |
| Coniferales | Douglas fir | *P. menziesii* | PME00064175 | 74% |
| | Scots pine | *P. sylvestris* | PPI00058992 | 71% |

[Table 2]

**Protein similarities in other species: Whole sequence similarity**

| Plant species | Scientific name | Transcript name | Protein similarity (%) |
|---|---|---|---|
| Rice | *O. sativa* | LOC_Os04g47240.1 | 100% |
| Common wheat | *T. aestivum* | Traes_2BL_23D01E7F4.1 | 98.6% |
| Brachypodium distachyon | *B. distachyon* | Brdisv1Tek-41020269m | 95.2% |
| Soybean | *G. max* | Glyma.11G158700.1 | 95.0% |
| Poplus | *P. deltoides* | Podel.T096600.1 | 93.3% |
| Barley | *H. vulgare* | HORVU2Hr1G096350.1 | 91.7% |
| Papaya | *C. papaya* | evm.TU.contig_45061.1 | 91.7% |
| Foxtail millet | *S. italica* | Seita.7G196800.1 | 90.8% |
| Pineapple | *A. comosus* | Aco014543.1 | 90.8% |
| Sorghum | *S. bicolor* | Sobic.006G176800.1 | 90.3% |
| Maize | *Z. mays* | ZmPHB47.02G070300.1 | 89.5% |
| Cabbage | *B. oleracea capitata* | Bol034534 | 88.9% |
| Domesticated Sunflower | *H. annuus* | HanXRQChr15g0472361 | 88.8% |
| Common Grape | *V. vinifera* | VIT_202s0025g03850.1 | 88.6% |
| Citrus | *C. clementina* | Ciclev10031888m | 85.5% |
| Tomato | *S. lycopersicum* | Solyc11g006000.1.1 | 85.1% |
| Cassava | *M. esculenta* | Manes.02G173500.1 | 83.8% |
| Thale cress | *A. thaliana* | AT1G63700.1 | 72.2% |
| Lettuce | *O. lettuca* | Lsat_1_v5_gn_8_7320.1 | 72.0% |
| Strawberry | *F.x ananassa* | maker-Fvb1-1-augustus-gene-118.33-mRNA-1 | 70.3% |

In a preferred embodiment, a plant of the next generation with controlled stomatal density is produced by regulating the methylation level of the promoter region of the YODA1 gene, among the genes involved in stomatal density. The promoters whose methylation levels are controlled are, in the case of rice, the P1 promoter, P2 promoter, and P3 promoter of OsYODA1. The relevant sequences are shown below.

OsYODA1 - P1 (SEQ ID NO: 2)

CAAAAGATGGTGGCTAGATGATCTGGGTTAAAAACCTCATCCCTTCTAATTAT
TTGATATTATATCATTCTCTAATATTCGCGTCTTTTTAAATCGATGAATGATGA
AATGACATCCCTTTTCTTTTGTGTAGCGAGTGAAAGTGTAGTACTT

OsYODA1 - P2 (SEQ ID NO: 3)

TCGGTTTAATTGTACACCGAATCACCAATTAAATTCAACTCTTTTATTTTTTT
TAGATTTTATAGATCCAACAAAAAAATTTATAGAGGAGTTCATCATAATTGCT
CTTTGACATTTTGTTAAAAAGTTAATCTAAAATGTTTGCCGTCGTACAATTGT
GAAAGGTCTATTTAGAACAAAGGTATTAAAACACAAAAATATTAGAAACATT
TCTCATACACAAATAATAGTAACGATAGGCATGCTGATGGCA

OsYODA1 - P3 (SEQ ID NO: 4)

AACGGACCGTCTCGATGAAGCTACCGTAGTCCGTAGCAACGGCGCCTTATATACAT
CCCCTCCATATCGAGGCTCGAAGCGTACTCGGTACTGCGGTAGCACGCCTGGGAA
CACAGAACACAGGGGGAAAAAAAGAACCGTTGCTACCCCGATGCGC

LsYODA1:promoter (Lettuce) (SEQ ID NO: 5)

TAACGACTTACCCCACTAGTACAAGTCCACTCTCTTCTCCTCCATTAAACAAATCTA
AAGTGAAAGCAAAAAAGAGAACCCAACCCCTCCTCCTCCTCTCTCTATACTTTCTC
CGATCTCTCTCTCTTGCTCTCCTTTTCCCAGTTTATTCTCACAGAACATCACTTGG
AAACCTCACAA

AtYODA1:promoter (*Arabidopsis*) (SEQ ID NO: 6)

GTGGTCAAAACACCAATGGGATAATAATAAAATGGTGTTTATATTGGGACTTTTCT
TCGTTTGTTGACCTATGTTGGGTCAACCAAGTATGATCTCTGAAGTGGTCAAAAGG
AGAGTTACTTTGTAATGTTGAACAAGAGCTTTTAAAAGAGTGATAGTGTGAGTGAG
TGTGCCTCTTGGTTTGTGGGAAGAAG

HvYODA1:promoter (Barley) (SEQ ID NO: 7)

AATCCCAAAGCCACGGGTCAACCAGACCCCAACCCGGTCCAGCACCGAGTTCCCT
CGAGCCTTGCAGTTTGCGGTTGGAAGGCAGAGGGGATCGATCCCGGCAGGCGAG
CCGCGGCGATCGTGTGGCGGTTGCCGCTTTGCCTCAGTTTCCACTGCTGTGTCTG
TGTGTGTGG

Promoters corresponding to the above promoters are also commonly present in plants other than rice, lettuce, *Arabidopsis thaliana*, and barley. Those skilled in the art can readily obtain the corresponding sequences from databases such as Phytozome (https://phytozome-next.jgi.doe.gov/) using the accession numbers listed in the above tables.

The promoter whose methylation level is controlled may be any of the following polynucleotides:

(a) a polynucleotide consisting of any one of the nucleotide sequences of SEQ ID NOS: 2 to 7;
(b) a polynucleotide consisting of a nucleotide sequence derived from any one of the nucleotide sequences of SEQ ID NOS: 2 to 7 by substitution, deletion, addition, or insertion of one or several nucleotides, and having a property of controlling expression of a gene operably linked downstream thereof;
(c) a polynucleotide consisting of a nucleotide sequence having a high sequence identity to any one of the nucleotide sequences of SEQ ID NOS: 2 to 7, and having a property of controlling expression of a gene operably linked downstream thereof.

[Environmental stress and temperature control during grain filling stage]

Examples of environmental stress include temperature stress (high temperature stress or low temperature

stress), salt stress, low light stress, high light stress, drought stress, waterlogging stress, heat stress, cold stress, nutritional stress (e.g., low nitrogen stress), heavy metal stress, disease stress, oxygen deficiency stress, ozone stress, $CO_2$ stress, and strong wind stress. Environmental stress is preferably temperature stress (high temperature stress or low temperature stress) during the seed growing (developing) period, or nutritional stress, such as low nitrogen stress. The step of exposing a plant to temperature stress can be performed by, for example, controlling the environmental temperature of the plant to a low or high temperature. The step of exposing a plant to nutritional stress, more specifically low nitrogen stress, can be performed by, for example, controlling the nitrogen content supplied to the plant to be low.

For the present invention, the term "grain filling stage" refers to the period during which the seeds grow, unless especially noted.

When the step of exposing a plant to environmental stress is performed by controlling the temperature during the grain filling stage, the temperature control during the grain filling stage can be performed by exposing the plant during the grain filling stage to a temperature higher or lower than the normal temperature for that plant during the grain filling stage. Regarding temperature control during the grain filling stage, a high temperature refers to a temperature higher than the reference temperature by, for example, 2 to 8°C or more, 3 to 7°C, or 4 to 6°C, and a low temperature refers to a temperature lower than the reference temperature by, for example, 2 to 8°C, 3 to 7°C, or 4 to 6°C.

In one preferred embodiment, when the plant is a *Poaceae* plant, the temperature control during the grain filling stage involves cultivating the plant for, for example, 6 weeks or longer after heading, at an environmental temperature at least 5°C higher than the normal average temperature for that period.

Preferred examples of the temperature control during the grain filling stage is as follows.

For wheat, set the environmental temperature during the grain filling stage to a temperature higher than 15°C, for example, 20 to 33°C, 22 to 32°C, or 25 to 31°C.

For barley, set the environmental temperature during the grain filling stage to a temperature higher than 15°C, for example, 20 to 33°C, 22 to 32°C, or 25 to 31°C.

For lettuce, set the environmental temperature during the grain filling stage to a temperature higher than 20°C, for example, 21 to 33°C, 22 to 32°C, 25 to 31°C, 27 to 40°C, 28 to 38°C, or 29 to 36°C.

For soybean, set the environmental temperature during the grain filling stage to a temperature higher than 20°C, for example, 21 to 33°C, 22 to 32°C, 25 to 31°C, 27 to 40°C, 28 to 38°C, or 29 to 36°C.

For corn, set the environmental temperature during the grain filling stage to a temperature higher than 20°C, for example, 21 to 40°C, 23 to 40°C, 25 to 40°C, 27 to 40°C, 28 to 38°C, or 29 to 36°C.

For strawberry, set the environmental temperature during the grain filling stage to a temperature higher than 10°C, for example, 11 to 33°C, 13 to 33°C, 15 to 33°C, 17 to 33°C, 18 to 28°C, 19 to 23°C, 21 to 34°C, 22 to 32°C, or 23 to 30°C.

In any case, the target plant is cultivated under the controlled temperature for one day or longer, three days or longer, one week or longer, three weeks or longer, or six weeks or longer.

[Methylation level]

In the present invention, the expression that the methylation level is effectively controlled means that methylation is attained at a degree sufficient to control the expression of downstream genes, unless especially noted. The degree of methylation may also be at a level that is higher or lower by than a certain percentage relative to the methylation level of a standard individual, if one exists.

The methylation level can be determined by measuring the degree of methylation in DNA extracted from the target seeds using an appropriate method. The measurement method is not particularly limited. There are (A) a method for analyzing methylated DNA after fragmenting and concentrating the methylated DNA; (B) an analysis method involving bisulfite treatment followed by sequencing; (C) an analysis method utilizing a methylation-sensitive restriction enzyme; (D) an analysis method utilizing methylation-specific PCR, etc., and any of these may be used.

A preferred example is a method comprising immunoprecipitation and concentration of 5-methylcytosine (5-mC) on single-stranded DNA (ssDNA) fragments using an antibody directed to 5-mC, which are performed for single-stranded DNA fragmented by sonication or with enzymes. For this measurement, a commercially available kit, such as MeDIP Kit, can be used.

The methylation level can be expressed as a percentage of input (% input), representing the proportion of methylated DNA relative to the total DNA used for analysis (input) (https://www.diagenode.com/files/products/kits/magmedip-qpcr-kit-manual.pdf).

Examples of the promoters and their preferred methylation levels are as follows.

P1 promoter of the rice OsYODA1 gene

Relative methylation level: 0.050% input or higher, preferably 0.060% input or higher, more preferably 0.065%

input or higher, further preferably 0.069% input or higher.

P2 promoter of the rice OsYODA1 gene

Relative methylation level: 0.020% input or higher, preferably 0.023% input or higher, more preferably 0.026% input or higher, further preferably 0.029% input or higher.

P3 promoter of the rice OsYODA1 gene

Relative methylation level: 0.012% input or higher, preferably 0.014% input or higher, more preferably 0.016% input or higher, further preferably 0.018% input or higher.

P1 promoter of lettuce LsYODA1 gene

Relative methylation level: 0.70% input or higher, preferably 1.00% input or higher, more preferably 1.40% input or higher, further preferably 1.80% input or higher.

Promoter of barley YODA1 gene

Relative methylation level: 0.055% input or higher, preferably 0.060% input or higher, more preferably 0.070% input or higher, further preferably 0.075% input or higher.

[Plant]

The plant to which the present invention is to be applied is not particularly limited. The present invention can be applied to both herbaceous and woody plants, and can be applied to both monocotyledonous and dicotyledonous plants. Examples of the plant include plants belonging to any order or family selected from *Amborellales*, *Nymphaeales*, *Austrobaileyales*, *Chloranthales*, *Canellales*, *Piperales*, *Laurales*, *Magnoliales*, *Acorales*, *Alismatales*, *Dioscoreales*, *Pandanales*, *Liliales*, *Asparagales*, *Dasypogonaceae*, *Arecales*, *Poales*, *Commelinales*, *Zingiberales*, *Ceratophyllales*, *Ranunculales*, *Sabiaceae*, *Proteales*, *Buxales*, *Trochodendrales*, *Gunnerales*, *Zygophyllales*, *Celastrales*, *Malpighiales*, *Oxalidales*, *Fabales*, *Rosales*, *Cucurbitales*, *Fagales*, *Geraniales*, *Myrtales*, *Crossosomatales*, *Sapindales*, *Huerteales*, *Brassicales*, *Malvales*, *Vitales*, *Saxifragales*, *Dilleniaceae*, *Berberidopsidales*, *Santalales*, *Caryophyllales*, *Cornales*, *Ericales*, *Garryales*, *Gentianales*, *Lamiales*, *Solanales*, *Aquifoliales*, *Apiales*, *Asterales*, and *Dipsacales*. Preferred examples of the plant include plants belonging to any order selected from *Poales*, *Brassicales*, *Malvales*, *Sapindales*, *Myrtales*, *Malpighiales*, *Fabales*, *Cucurbitales*, *Rosales*, *Vitales*, *Asterales*, *Lamiales*, *Solanales*, *Caryophyllales*, *Ranunculales*, *Zingiberales*, and *Coniferales* (*Pinales*, substantially corresponding to *Pinophyta*).

Examples of plants suitable for the application of the present invention include rice (*O. sativa*), watercress (*A. thaliana*), turnip mustard (*B. rapa*), papaya (*C. papaya*), cacao (*T. cacao*), Orange (*C. sinensis*), eucalyptus (*E. grandis*), poplar (*P. trichocarpa*), soybean (*G. max*), cowpea (*V. unguiculata*), cucumber (*C. sativus*), strawberry (*F.* x *ananassa*), grape (V. vinifera), lettuce (*L. sativa*), sunflower (*H. annuus*), olive (*O. europaea*), tomato (*S. lycopersicum*), amaranth (*A. hypochondriacus*), sugar beet (*B. vulgaris*), spinach (*S. oleracea*), Colorado columbine (*A. coerulea*), corn (*Z. mays*), wheat (*T. aestivum*), barley (*H. vulgare*), banana (*M. acuminata*), Douglas fir (*P. menziesii*), and scots pine (*P. sylvestris*).

For the present invention, the term "plant" is used in the sense of an individual plant or a part thereof, unless especially indicated, and the term "part thereof" encompasses seed (including germinated seed and immature seed), organ or a part thereof (including leaf, root, stem, flower, stamen, pistil, and fragments thereof), cultured plant cells, callus, and protoplast, unless especially indicated. The plant may be a genetically engineered plant or transformed plant. The plant may be a harvested product or propagation material. For the present invention, the terms propagation material refers to all or a part of a plant body that is used for propagation (sometimes referred to as seedling), unless especially noted, and examples include, for example, seed, seedling (nursery stock and shrub), scion, bulb, cell, callus, plumule etc. For the present invention, the term "harvested product" is used in the usual sense, unless especially noted, and may be all or a part of a plant body that is not used for propagation. For example, if the plant is rice, the harvested product encompasses harvested rice and paddy. The harvested product may also be referred to as crop or agricultural crop. For the present invention, the term "processed product" refers to a processed product that is produced directly or indirectly from a harvested product, unless especially noted. For the present invention, the processed product encompasses processed products that reflect characteristics of the harvested product obtained according to the present invention. For example, when the plant is rice, milled rice, cocked rice, rice flour, breads, confectioneries, buns, and pizza using rice flour, and liquor (sake) are included in the scope of the processed product.

For the present invention, the term "progeny" used for a plant means a plant of which at least one of genetic parent

and/or ancestor is the plant in question, unless especially noted. The progeny may be a progeny obtained from a transformed plant as the parent (T1 etc.) and a progeny thereof, as well as a crossed progeny of which one of the parent is T0 or T1 (e.g., F1) and a progeny thereof, as long as the desired trait is inherited. The first generation of progeny may be referred to as the next generation. Progeny include the next generation, next-next generation, and so on.

[Plants with increased stomatal density and use thereof]

The present invention also provides a plant with increased stomatal density, which is obtainable by the production method described above, seed or seedling thereof, and processed product thereof. The plant with increased stomatal density provided by the present invention has useful characteristics. For the present invention, examples of the useful characteristics include increased biomass, improved carbon dioxide fixation ability, and high temperature tolerance.

The plant with increased stomatal density exhibit enhanced carbon dioxide absorption, resulting in superior ability to fix atmospheric carbon dioxide and enabling fixation of greater amounts of carbon dioxide. Further, the plant with increased stomatal density show increased carbon fixation due to the increased carbon dioxide absorption, potentially leading to increased yields. Furthermore, the plant with increased stomatal density can absorb more materials (fertilizers, e.g., the three major nutrients, nitrogen, phosphorus and potassium; secondary and micronutrients; chemical agents such as pesticides, etc.) through the leaf surfaces thereof, and this allows reduced application amounts of such materials, potentially contributing to the production of crops with lower environmental impact.

As plants used for such purposes, vascular plants are preferred because the effects of increased stomatal density are significant. Vascular plants are not particularly limited, but examples include *Fabaceae* plants represented by soybean, *Poaceae* plants represented by rice, *Brassicaceae* plants represented by *Arabidopsis thaliana*, *Salicaceae* plants represented by black cottonwood etc., and *Selaginellaceae* plants represented by *Selaginella moellendorffii* Hieron.

## Examples

<Rice>

[Materials and Methods]

1. Plant Material, growing conditions, and growth measurement

Rice plants (*Oryza sativa L.* cv. Nipponbare) were cultivated under natural conditions. The cultivation sites were Kyushu University Hakozaki Campus (Fukuoka, Japan, 33°67’ N, 130°42’ E) during 2015 to 2018 and Kyushu University Ito Campus (Fukuoka, Japan, 33°37’ N, 130°25’ E) during 2019 to 2020. Rice plants were grown under control conditions and subjected to a heat stress treatment during the grain filling stage, as previously reported (Suriyasak et al. 2020). For growth measurements, one 3-week old control rice seedling and one 3-week old rice seedling obtained from a seed ripened at a high temperature were transplanted into 1/5000 Wagener’s pots with base fertilizer. The base fertilizer was as described in the previous study (Suriyasak et al. 2020).

2. Analyses of stomatal density, stomatal conductance, and transpiration rate; analysis of biomass under high $CO_2$ conditions; and analysis of photosynthetic rate

Rice plants (*Oryza sativa L.* cv. Nipponbare) were sown under conditions of 400 ppm or 800 ppm $CO_2$ (also at 25°C), and sampling was performed three weeks after sowing. Photosynthetic rate analysis was conducted according to the method described in the literature (Egashira et al. 2020).

3. Comprehensive DNA methylation analysis by whole genome bisulfite sequencing (WGBS) method

Genomic DNA from each sample was extracted from 20 dried seeds using the ISOSPIN plant DNA extraction kit (Nippon Gene). Pooled gDNA extracted from 10 samples derived from each treatment was subjected to a bisulfite treatment prior to the sequence analysis. Reference genome sequence, genes and TE (transposable element) annotations were obtained from RAP-DB. Those with a methylation level that differed by 1.25-fold or more (difference in methylation level between the control and treatment plot did not fall within the range of ±25%, P value was less than 0.05) were extracted as differentially methylated positions (DMPs) and differentially methylated regions (DMRs), and the extracted DMRs were screened for 100 bp at a time using the sliding window method. DMRs located within 3000 bp upstream and downstream of the translation region, respectively, were considered as transcription start sites (TSS) of the promoter. Gene ontology (GO) analysis was performed by using the "GO biological process complete", "GO molecular

function complete" and "GO cellular component complete" analysis set of Panther Software and the setting of 'Fisher's Exact' test with 'No correlation'. The genes involved in each GO are listed in the table in descending order of number.

4. RNA Sequence analysis and quantitative real-time PCR

Seedlings at 18 days after sowing were used for RNA sequence analysis. All the differentially expressed genes (DEGs, P < 0.05) were analyzed, and a heatmap of RNA reads was generated by using OriginPro. GO analysis for DEGs was performed by using the same method as described above for DMR. For quantitative real-time PCR (qRT-PCR), cDNA was synthesized from the extracted RNA by using the ReverTra Ace reverse transcriptase (Toyobo) according to the manufacturer's instructions. The quantitative real-time PCR was performed by using the CFX Connect Optics Module real-time PCR detection system (Bio-Rad) with SYBR Green (Toyobo) according to the manufacturer's instructions. The primers used for qRT-PCR are listed in Supplemental Table S5. The PCR conditions were as follows. Initial denaturation was performed at 94°C for 2 minutes, followed by 40 cycles of denaturation at 94°C for 20 seconds, annealing at the temperature set for each primer for 20 seconds, and extension at 72°C for 20 seconds. Thereafter, melting curve analysis was performed, and data from the plate were analyzed. The results were normalized based on the expression levels of OsUBQ (leaf samples) or OsActin (root and developing grain samples).

5. DNA methylation analysis by MeDIP-qPCR

Genomic DNA was extracted from dry seeds and seedlings using the DNeasy Plant Mini Kit (Qiagen) and sheared into approximately 500 bp fragments by sonication. For MeDIP-qPCR, immunoprecipitation was performed with 1000 ng of the sheared DNA using an immunoprecipitation kit (Active Motif) as described in the manufacturer's protocol. The immunoprecipitated DNA was subjected to qRT-PCR to detect locations of changes in DNA methylation level. Percent input was calculated as described in the manufacturer's protocol (https://www.diagenode.com/files/products/kits/mag medip-qpcr-kit-manual.pdf). Specifically, % input is a ratio of DNA precipitated with the methylation antibody directed to the DNA used in the analysis (input).

[Results]

1. High temperature stress during the grain filling stage causes phenotypic changes in progeny plants, resulting in increased stomatal density.

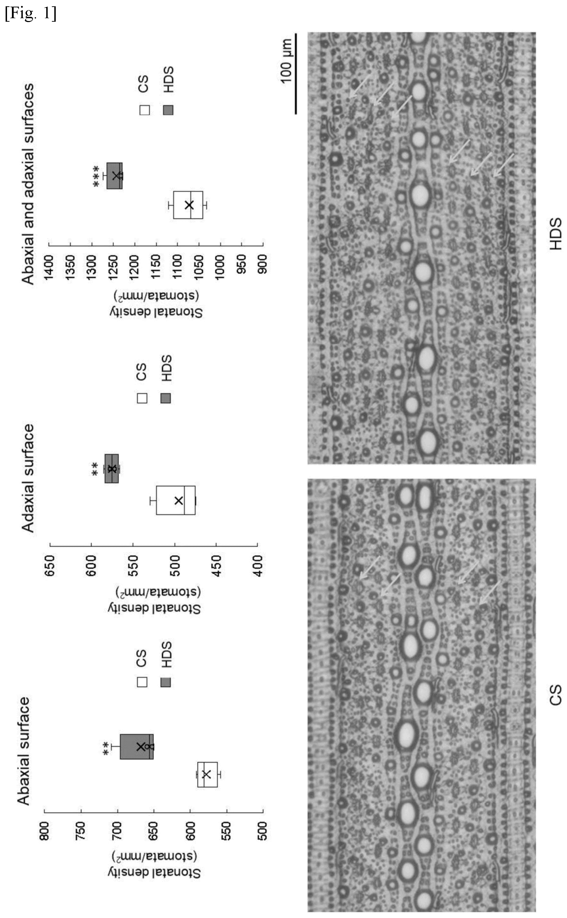

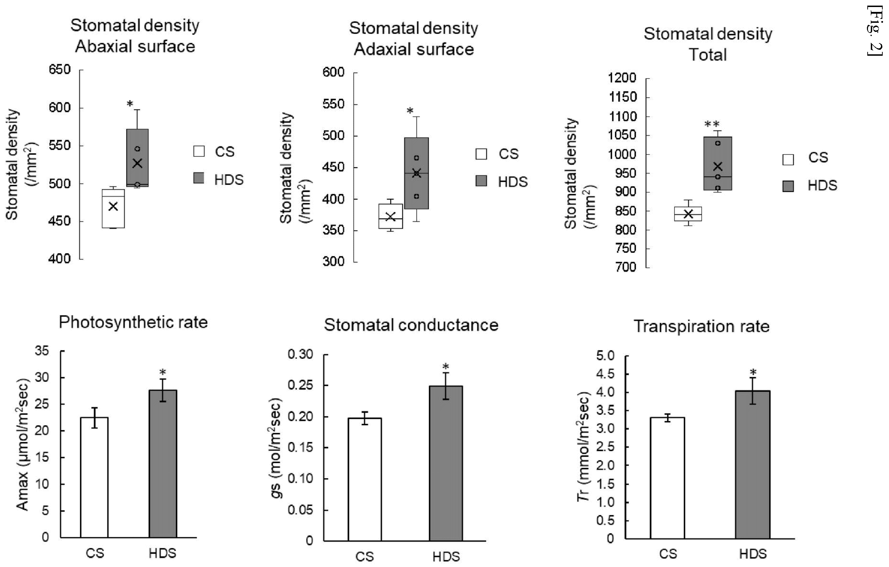

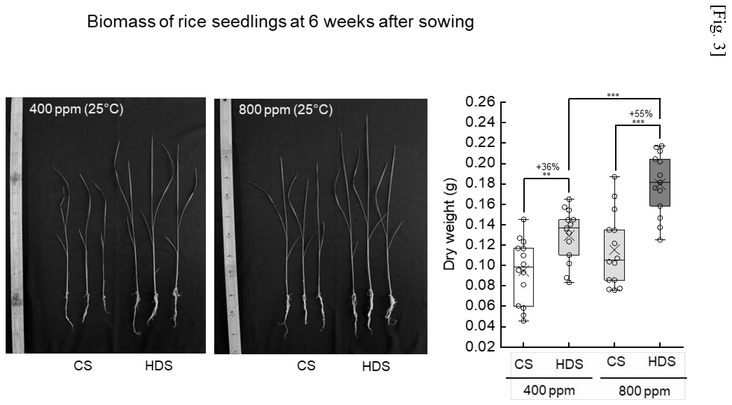

To clarify how high temperature stress during the grain filling stage affects phenotypic changes in progeny, seeds from control plants (grain filling at 22 to 32°C) and plants subjected to high temperature stress (grain filling at 28 to 38°C) were sown and grown under natural environmental conditions. As a result, in the field test, plants grown from seeds ripened at high temperatures showed significantly increased stomatal densities on both the adaxial and abaxial surfaces compared with the control (Fig. 1). Further, also in the pot test, plants grown from seeds ripened at high temperatures also showed significantly increased stomatal densities, stomatal conductances, and transpiration rates compared with the control (Fig. 2). Furthermore, in the cultivation under high $CO_2$ conditions, plants grown from seeds ripened at high temperatures exhibited significantly higher biomasses at 6 weeks after sowing compared with the control. Notably, when comparing plants grown from seeds ripened at high temperatures under $CO_2$ levels of 400 ppm versus 800 ppm, the latter showed significantly higher biomasses (Fig. 3). Plants grown from seeds ripened at high temperatures were found to be capable of fixing more $CO_2$ under high $CO_2$ conditions.

2. High-temperature stress during the grain filling stage induces comprehensive methylome changes in dry seeds

The inventors of the present invention hypothesized that epigenetic control, in particular, DNA methylation, plays a crucial role in this intergenerational memory, and methylomes were generated by comparing methylated cytosines at the single-base level between control seeds and seeds ripened with high temperature stress. Comprehensive methylation analysis by whole-genome bisulfite sequencing (WGBS) revealed that, in the control seeds, 56.6%, 27.0%, and 14.1% of cytosine residues were methylated in the CG, CHG, and CHH contexts, respectively, but in the seeds ripened with high temperature stress, 57.8%, 28.5%, and 15.5% of total 3,991,072,994 sequenced cytosines were methylated in the CG, CHG, and CHH contexts, respectively (see the following table).

[Table 3]

Total cytosines analyzed by whole genome bisulfite sequencing

| | Control | Heat |
|---|---|---|
| Total cytosines analysed | 3,919,488,890 | 3,991,072,994 |
| Duplication rate (%) | 12.1% | 10.8% |
| Coverage cytosine for mapping (%) | 97.9% | 97.9% |
| Methylated cytosines in CpG context | 356,642,852 | 369,500,795 |
| Methylated cytosines in CHG context | 166,128,581 | 177,195,999 |
| Methylated cytosines in CHH context | 375,821,081 | 423,437,019 |
| Unmethylated cytosines in CpG context | 273,533,402 | 270,050,603 |
| Unmethylated cytosines in CHG context | 449,808,489 | 443,743,999 |
| Unmethylated cytosines in CHH context | 2,297,554,485 | 2,307,144,579 |
| CpG methylation (%) | 56.6% | 57.8% |
| CHG methylation (%) | 27.0% | 28.5% |
| CHH methylation (%) | 14.1% | 15.5% |

In summary, high temperature stress during the grain filling stage induced approximately 1% hypermethylation in all the methylation contexts. When plotting the average methylation levels on the chromosome for all the contexts as a chromosome map using 1 Mbp window sizes, seeds ripened with high temperature stress showed genome-wide changes in methylation levels compared with the control. Then, differentially methylated regions (DMRs) in seeds ripened at high temperatures compared with the control were identified, and as a result, 268 hypermethylated DMRs and 189 hypomethylated DMRs, 457 DMRs in total, were identified. Analysis of DMR distribution revealed that the majority of DMRs in the CG context overlapped with gene promoters, and both hyper- and hypomethylated DMRs secondly frequently occurred in the transposable element (TE) regions.

In contrast, in the non-CG context, most DMRs were located in the TE regions, and secondly in the promoter regions. It was further found that DMRs overlapping with TEs were most abundant in retrotransposons. GO analysis of DMRs overlapping with protein-coding genes revealed a rich presence of DMRs associated with stress response, metal ion transport, oxidative stress response, and various biological processes.

These results suggested that exposure to high temperature stress during the grain filling stage induces comprehensive methylome changes especially in the promoter regions of coded genes.

3. High temperature stress during the grain filling stage induces transcriptional changes during the vegetative growth of progeny.

To obtain deeper insight into how comprehensive DNA methylation changes during plant development affect transcriptional regulation, RNA sequencing was performed by using RNA from leaves of progeny in the vegetative growth stage.

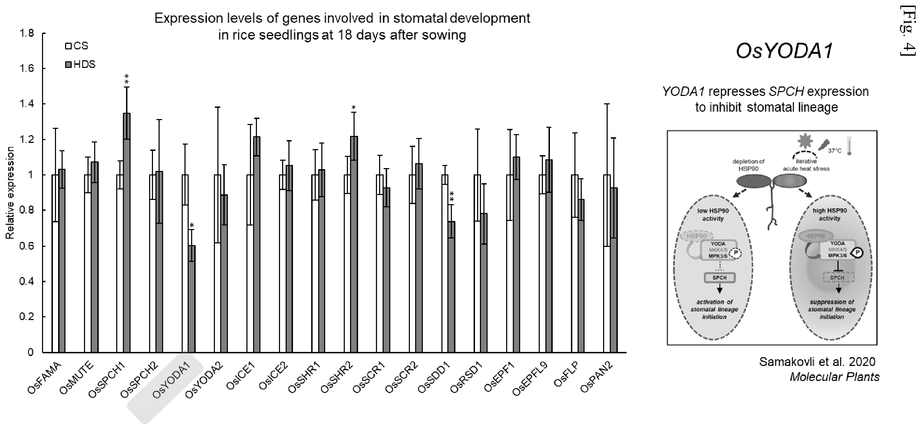

As a result, reduced expression of OsYODA1 (SEQ ID NO: 1) was observed in the progeny, among the genes involved in the stomatal development (Fig. 4). YODA1 is known to suppress SPCH expression and inhibit stomatal formation (Samakovli et al. 2020, Molecular Plants).

These findings suggest that reduced OsYODA1 expression may play a crucial role in the stomatal density phenotype observed in progeny plants grown from seeds subjected to high temperature stress during the grain filling stage.

4. Changes in DNA methylation in dry seeds are maintained in growing organs of progeny and involved in transcriptional regulation.

The results obtained thus far indicated that changes in OsYODA1 expression during progeny plant growth in response to high temperature stress are associated with stomatal density, and it was anticipated that changes in DNA methylation levels at the promoters of the gene may play a crucial role in transcriptional regulation. Therefore, the DNA methylation levels of the OsYODA1 gene promoters were analyzed in both dry seeds and growing organs of the progeny by MeDIP-qPCR (methylated DNA immunoprecipitation-qPCR) using locus-specific amplicons.

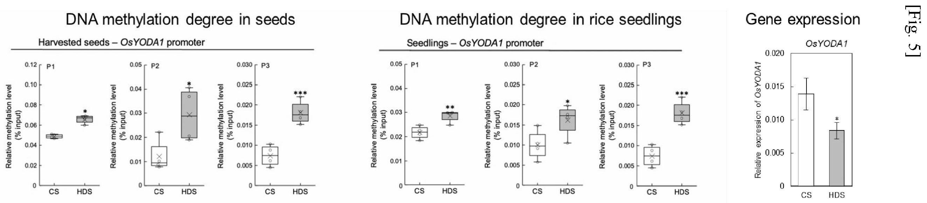

As a result, it was shown that the promoter regions of OsYODA1 (P1 (SEQ ID NO: 2), P2 (SEQ ID NO: 3), and P3 (SEQ ID NO: 4)) were significantly hypermethylated in both the dry seeds of the plants subjected to high temperature stress and seedlings obtained therefrom (Fig. 5).

In summary, these data suggest that exposure to high temperature stress during the grain filling stage induces hypermethylation of the OsYODA1 gene promoters, leading to the increased stomatal density phenotype, and further, a part of this DNA methylation state is maintained from dry seeds to growing organs in the progeny.

[Discussion]

The results of this study indicate that if parental plants experienced high temperature stress during the grain filling stage, an increase in stomatal density occurs in progeny rice plants compared with the control.

The results of this study indicate that exposure to high temperature stress during the grain filling stage induces significant hypomethylation of the OsYODA1 gene promoters in both dry seeds and leaves of the progeny, resulting in reduced expression of the OsYODA1 gene.

Methylation in gene promoter regions is known to be associated with transcriptional regulation (Zhang et al. 2006). In this study, the highest number of DMRs was found in gene regions, especially promoters, suggesting they may contribute to various transcriptional changes during growing. Further, DMRs were also observed in transposable element (TE) regions, especially retrotransposons, which are known to influence gene expression in response to various stimuli (Galindo-Gonzalez et al. 2017). On the basis of these results, this study proposes a role of DNA methylation in intergenerational memory in rice, but further clarification is needed regarding other epigenetic marks, such as TE regulation and histone modifications.

In conclusion, this study proposes that high temperature stress during the grain filling stage significantly influences phenotypic changes in progeny plants through comprehensive DNA methylation occurring in seeds, and a part of this methylation is maintained at specific loci in growing organs and participates in transcriptional regulation. Among the observed phenotypic changes, the increase in stomatal density and the resulting improvement in $CO_2$ fixation represent advantageous traits for rice production and are expected to provide substantial benefits to humanity.

<Lettuce, *Arabidopsis thaliana*, barley, wheat, soybean, corn, and strawberry>

[Materials and Methods]

Monocotyledons, barley (*Hordeum vulgare* L. cv. Ichibanboshi) and wheat (*Triticum aestivum* L. cv. Shiroganekomugi), and dicotyledons, *Arabidopsis thaliana* (*Arabidopsis thaliana* accession Col-0), lettuce (*Lactuca sativa* cv. Chima Sanchu), and soybean (*Glycine max* (L.) Merr. cv. Fukuyutaka), were cultivated in the same manner as the method used for rice. In addition, corn (*Zea mays* L. cv. P2088) and strawberry (*Fragaria* x *ananassa* cv. Toyonoka) were also cultivated.

[Results]

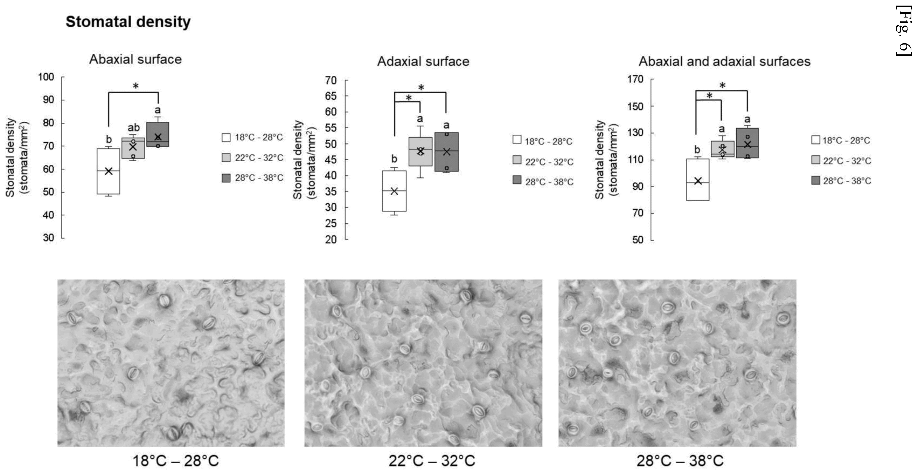

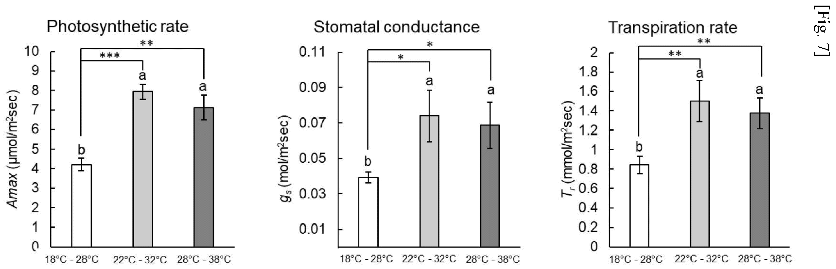

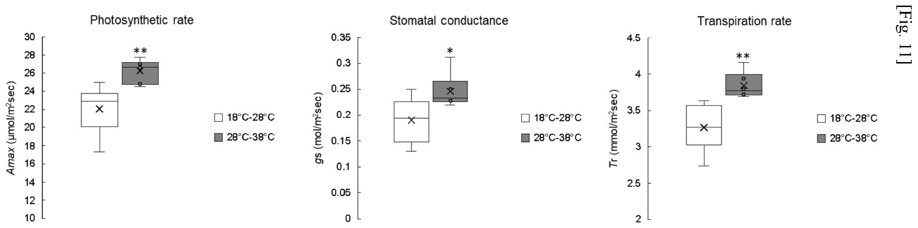

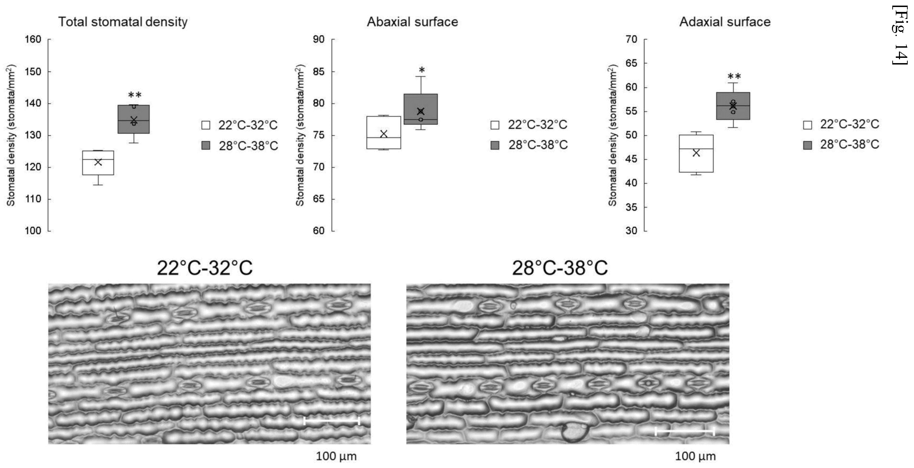

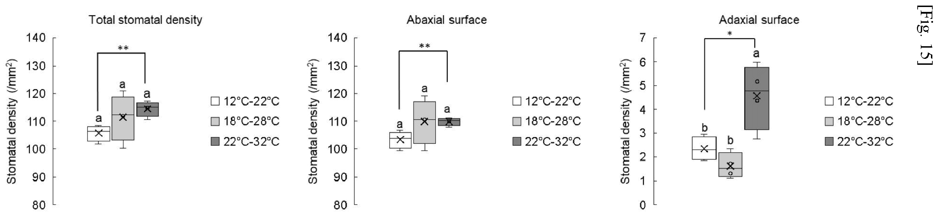

The obtained results are shown in Figs. 6 to 15, along with the cultivation and experimental conditions. It was demonstrated that the density of stomata can be controlled by regulating the temperature during the grain filling stage in both monocotyledons and dicotyledons.

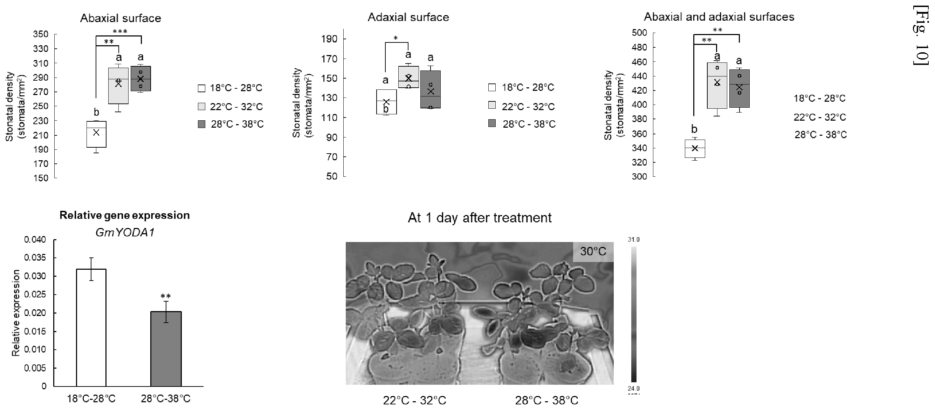

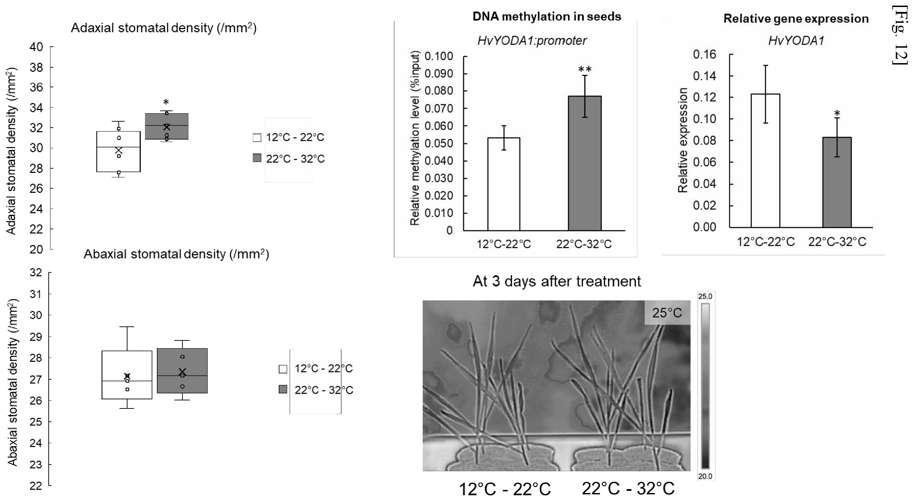

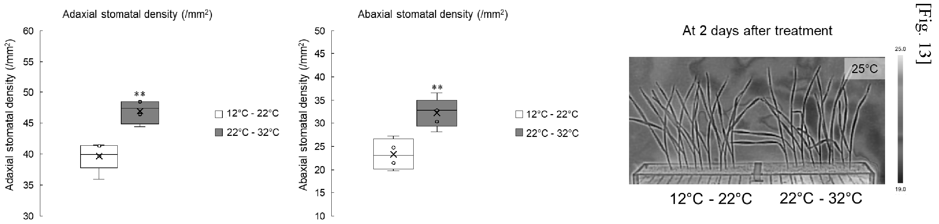

For barley, wheat, and soybean, the effects were analyzed after 3 days, 2 days, and 1 day from the treatment, respectively, by using FLIR C2 thermography camera (FLIR Systems AB, Sweden). As a result, it was observed that the temperatures around the progeny plants with increased stomatal density were lower (Fig. 10, bottom right; Fig. 12, bottom right; Fig. 13, bottom right), indicating higher transpiration and enhanced heat tolerance.

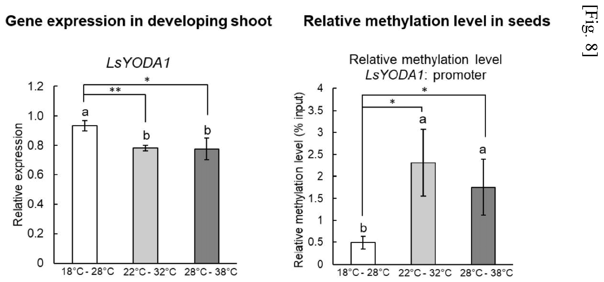

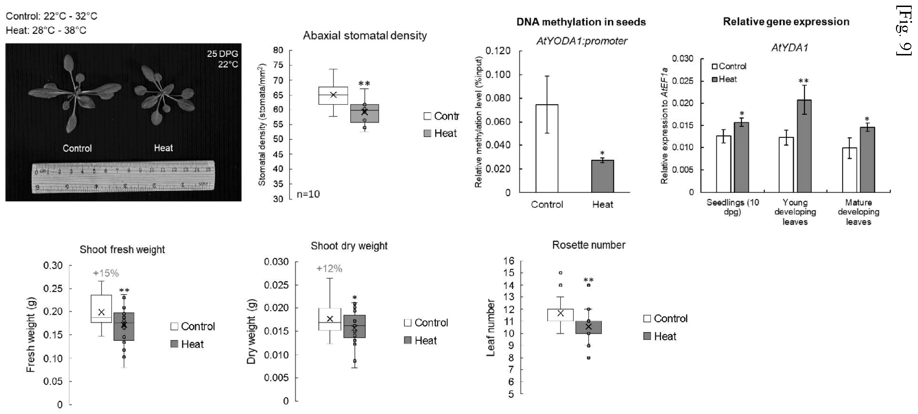

For lettuce and barley, increased relative methylation levels were observed in the promoter regions of the YODA1 gene (SEQ ID NOS: 5 and 7), and reduced YODA1 gene expression could be confirmed (Figs. 8 and 12). For *Arabidopsis thaliana*, the relative methylation level of the promoter region of the YODA1 gene (SEQ ID NO: 6) decreased, and increased expression of the YODA1 gene could be confirmed (Fig. 9).

<References cited in the section of Examples >

1) Suriyasak, C. et al. Mechanism of delayed seed germination caused by high temperature during grain filling in rice (Oryza sativa L.). Sci. Rep. 10, 17378 (2020) (Non-patent document 2 cited above)

2) Zhang, X. et al. Genome-wide-high-resolution mapping and functional analysis of DNA methylation in Arabidopsis. Cell 126, 1189-1201 (2006).

3) Galindo-Gonzalez, L. et al. LTR retrotransposons in plants: Engines of evolution. Gene 626, 14-25 (2017).

4) Egashira et al. A rapid translocation of photoassimilates from source organs maintains grain yield in cowpea

subjected to drought stress during grain filling. Biologia plantarum 64:529-534 (2020).
5) Samakovli et al. Molecular Plants YODA-HSP90 Module Regulates Phosphorylation-Dependent Inactivation of SPEECHLESS to Control Stomatal Development under Acute Heat Stress in Arabidopsis. Molecular Plant 13, 612-633 (2020).

<Sequence listed in Sequence Listing>

SEQ ID NO: 1, OsYODA1 amino acid sequence
SEQ ID NO: 2, OsYODA1 P1 nucleotide sequence
SEQ ID NO: 3, OsYODA1 P2 nucleotide sequence
SEQ ID NO: 4, OsYODA1 P3 nucleotide sequence
SEQ ID NO: 5, LsYODA1: promoter (lettuce) nucleotide sequence
SEQ ID NO: 6, AtYODA1: promoter (Arabidopsis) nucleotide sequence
SEQ ID NO: 7, HvYODA1: promoter (barley) nucleotide sequence

Industrial Applicability

The present invention enables the production of plants with controlled stomatal density, which is useful in the fields of agriculture, industrial crop production etc. Plants with increased stomatal density can absorb more materials through their leaves, potentially reducing the amounts of materials required for application, and contributing to the production of crops with a lower environmental impact. Further, plants with increased stomatal density can fix more carbon dioxide from the atmosphere, and therefore the present invention is expected to have applications such as increasing carbon dioxide absorption by forests.

## Claims

1. A method for producing a progeny plant with controlled stomatal density, the method comprising the step of cultivating a plant under environmental stress.

2. The method according to claim 1, wherein the step of cultivating a plant under environmental stress comprises the step of controlling the temperature during the grain filling stage of the plant.

3. The method according to claim 1, wherein the control of stomatal density is achieved by controlling methylation of a promoter region of stomatal development related genes.

4. The method according to claim 1, wherein the control of stomatal density is achieved by controlling methylation of a promoter region of a gene encoding any one of the following proteins:

   (A) a protein consisting of the amino acid sequence of SEQ ID NO: 1;
   (B) a protein consisting of an amino acid sequence derived from the amino acid sequence of SEQ ID NO: 1 by substitution, deletion, addition, or insertion of one or several amino acids, provided that the portion corresponding to the portion consisting of the amino acids at positions 236 to 330 is identical, and having a property of controlling stomatal formation in plants; and
   (C) a protein consisting of an amino acid sequence having a sequence identity of 60% or higher to the amino acid sequence of SEQ ID NO: 1, provided that the portion corresponding to the portion consisting of the amino acids at positions 236 to 330 is identical, and having a property of controlling stomatal formation in plants.

5. The method according to claim 4, wherein expression of the gene is reduced by controlling methylation of the promoter region of the gene, thereby producing a progeny plant with increased stomatal density.

6. A plant with increased stomatal density obtainable by the method according to claim 5, a seed, a seedling, or a processed product thereof.

7. A method for fixing atmospheric carbon dioxide using the plant according to claim 6.

**8.** A method for cultivating the plant according to claim 6, the method comprising the step of applying a material by foliar spray.

**9.** A method for controlling stomatal density of a progeny plant, the method comprising the step of cultivating a plant under environmental stress.

**10.** The method according to claim 9, wherein the step of cultivating a plant under environmental stress comprises the step of controlling the temperature during the grain filling stage of the plant.

[Fig. 1]

Abaxial surface
Stomatal density (stomata/mm²)
800
750
700
650
600
550
500
**
CS
HDS
Adaxial surface
Stomatal density (stomata/mm²)
650
600
550
500
450
400
**
CS
HDS
Abaxial and adaxial surfaces
Stomatal density (stomata/mm²)
1400
1350
1300
1250
1200
1150
1100
1050
1000
950
900
***
CS
HDS
100 µm
CS
HDS

[Fig. 2]

Stomatal density
Abaxial surface
Stomatal density
Adaxial surface
Stomatal density
Total
CS
HDS
Stomatal density (/mm²)
Photosynthetic rate
Amax (µmol/m²sec)
Stomatal conductance
gs (mol/m²sec)
Transpiration rate
Tr (mmol/m²sec)

[Fig. 3]

Biomass of rice seedlings at 6 weeks after sowing
400 ppm (25°C)
800 ppm (25°C)
CS
HDS
CS
HDS
Dry weight (g)
0.26
0.24
0.22
0.20
0.18
0.16
0.14
0.12
0.10
0.08
0.06
0.04
0.02
+36%
**
+55%
***
***
CS
HDS
CS
HDS
400 ppm
800 ppm

[Fig. 4]

Expression levels of genes involved in stomatal development in rice seedlings at 18 days after sowing
□CS
■HDS
Relative expression
0
0.2
0.4
0.6
0.8
1
1.2
1.4
1.6
1.8
OsFAMA
OsMUTE
OsSPCH1
OsSPCH2
OsYODA1
OsYODA2
OsICE1
OsICE2
OsSHR1
OsSHR2
OsSCR1
OsSCR2
OsSDD1
OsRSD1
OsEPF1
OsEPFL9
OsFLP
OsPAN2
OsYODA1
YODA1 represses SPCH expression to inhibit stomatal lineage
depletion of HSP90
iterative acute heat stress
37°C
low HSP90 activity
high HSP90 activity
HSP90
YODA
MKK4/5
MPK3/6
SPCH
P
activation of stomatal lineage initiation
suppression of stomatal lineage initiation
Samakovli et al. 2020
Molecular Plants

[Fig. 5]

DNA methylation degree in seeds
Harvested seeds – OsYODA1 promoter
Relative methylation level (% input)
P1
P2
P3
CS
HDS
DNA methylation degree in rice seedlings
Seedlings – OsYODA1 promoter
Gene expression
OsYODA1
Relative expression of OsYODA1

[Fig. 6]

Stomatal density
Abaxial surface
Adaxial surface
Abaxial and adaxial surfaces
Stonatal density (stomata/mm²)
18°C - 28°C
22°C - 32°C
28°C - 38°C
18°C – 28°C
22°C – 32°C
28°C – 38°C

[Fig. 7]

Transpiration rate
$T_r$ (mmol/m²sec)
2
1.8
1.6
1.4
1.2
1
0.8
0.6
0.4
0.2
0
**
**
b
a
a
18°C - 28°C
22°C - 32°C
28°C - 38°C

Stomatal conductance
$g_s$ (mol/m²sec)
0.11
0.09
0.07
0.05
0.03
0.01
*
*
b
a
a
18°C - 28°C
22°C - 32°C
28°C - 38°C

Photosynthetic rate
$Amax$ (μmol/m²sec)
10
9
8
7
6
5
4
3
2
1
0
***
**
b
a
a
18°C - 28°C
22°C - 32°C
28°C - 38°C

[Fig. 8]

Relative methylation level in seeds
Relative methylation level
LsYODA1: promoter
Relative methylation level (% input)
0
0.5
1
1.5
2
2.5
3
3.5
4
18°C - 28°C
22°C - 32°C
28°C - 38°C
a
b
*

Gene expression in developing shoot

LsYODA1
Relative expression
0
0.2
0.4
0.6
0.8
1.0
1.2
18°C - 28°C
22°C - 32°C
28°C - 38°C
a
b
**
*

[Fig. 9]

Control: 22°C - 32°C
Heat: 28°C - 38°C
25 DPG
22°C
Control
Heat
Abaxial stomatal density
Stomatal density (stomata/mm²)
n=10
Contr
Heat
DNA methylation in seeds
AtYODA1:promoter
Relative methylation level (%input)
Control
Heat
Relative gene expression
AtYDA1
Control
Heat
Relative expression to AtEF1a
Seedlings (10 dpg)
Young developing leaves
Mature developing leaves
Shoot fresh weight
Fresh weight (g)
+15%
Control
Heat
Shoot dry weight
Dry weight (g)
+12%
Control
Heat
Rosette number
Leaf number
Control
Heat

[Fig. 10]

Abaxial surface
Stomatal density (stomata/mm²)
18°C - 28°C
22°C - 32°C
28°C - 38°C
Adaxial surface
Stomatal density (stomata/mm²)
18°C - 28°C
22°C - 32°C
28°C - 38°C
Abaxial and adaxial surfaces
Stomatal density (stomata/mm²)
18°C - 28°C
22°C - 32°C
28°C - 38°C
Relative gene expression
GmYODA1
Relative expression
18°C-28°C
28°C-38°C
At 1 day after treatment
30°C
22°C - 32°C
28°C - 38°C

[Fig. 11]

Photosynthetic rate
Amax (μmol/m²sec)
30
28
26
24
22
20
18
16
14
12
10
**
18°C-28°C
28°C-38°C
Stomatal conductance
gs (mol/m²sec)
0.35
0.3
0.25
0.2
0.15
0.1
0.05
0
*
18°C-28°C
28°C-38°C
Transpiration rate
Tr (mmol/m²sec)
4.5
4
3.5
3
2.5
2
**
18°C-28°C
28°C-38°C

[Fig. 12]

Adaxial stomatal density (/mm²)
Adaxial stomatal density (/mm²)
40
38
36
34
32
30
28
26
24
22
20
*
12°C - 22°C
22°C - 32°C
Abaxial stomatal density (/mm²)
Abaxial stomatal density (/mm²)
32
31
30
29
28
27
26
25
24
23
22
12°C - 22°C
22°C - 32°C
DNA methylation in seeds
HvYODA1:promoter
Relative methylation level (%input)
0.100
0.090
0.080
0.070
0.060
0.050
0.040
0.030
0.020
0.010
0
**
12°C-22°C
22°C-32°C
Relative gene expression
HvYODA1
Relative expression
0.16
0.14
0.12
0.10
0.08
0.06
0.04
0.02
0.00
*
12°C-22°C
22°C-32°C
At 3 days after treatment
25°C
25.0
20.0
12°C - 22°C
22°C - 32°C

[Fig. 13]

At 2 days after treatment
25°C
12°C - 22°C
22°C - 32°C
Abaxial stomatal density (/mm²)
Abaxial stomatal density (/mm²)
□ 12°C - 22°C
■ 22°C - 32°C
**
Adaxial stomatal density (/mm²)
Adaxial stomatal density (/mm²)
□ 12°C - 22°C
■ 22°C - 32°C
**

[Fig. 14]

Total stomatal density
Stomatal density (stomata/mm²)
160
150
140
130
120
110
100
**
22°C-32°C
28°C-38°C
Abaxial surface
Stomatal density (stomata/mm²)
90
85
80
75
70
65
60
*
22°C-32°C
28°C-38°C
Adaxial surface
Stomatal density (stomata/mm²)
70
65
60
55
50
45
40
35
30
**
22°C-32°C
28°C-38°C
22°C-32°C
100 µm
28°C-38°C
100 µm

[Fig. 15]

Total stomatal density
Abaxial surface
Adaxial surface
Stomatal density (/mm²)
12°C-22°C
18°C-28°C
22°C-32°C
**
*
a
b
140
130
120
110
100
90
80
7
6
5
4
3
2
1
0

[Fig. 16]

OsYODA1(OsMPKKK10) SEQ ID NO:1

MPPWWGKSFSKDAKKTTKENLIDTFHRLISPNDQKGSTKSKRSCRRGNDSSVEKSCRSTTVS
RPTSPSKEVSRCQSFSADRPHAHPLPIPGVRPPVTRTVSDITESKPILEKRGKPPLLLPLPKPNR
PPRRHGNSEVVSEIVVASPSSNCSDSDDHGDSQLQSPVGNDAENATLVTLKNKSSNARKECP
GPITAKNMKEIHRPANQVHGSHILSTSPRGVAADSYQSNLQNPRPLVLDSAPNSLMSSPSRSP
RRICPDHIPTSAFWAVKPHTDVTFVGSGQCSSPGSGQTSGHNSVGGDMLAQLFWQPSRSSP
ECSPIPSPRMTSPGPSSRVHSGSVSPLHPRSGGMAPESPTNRHDDGKKKQTHKLPLPPLSIS
HSSFHPNNSTPTSPISVPRSPGRTENPPSPGSRWKKGKLIGRGTFGHVYVGFNSDSGEMCAM
KEVTLFLDDPKSKESAKQLGQEISLLSRLQHPNIVQYYGSETVDDKLYIYLEYVSGGSIHKLLQE
YGQLGEQAIRSYTQQILSGLAYLHAKNTVHRDIKGANILVDPSGRVKLADFGMAKHINGQQCPF
SFKGSPYWMAPEVIKNSNGCNLAVDIWSLGCTVLEMATSKPPWSQYEGIAAMFKIGNSKELPP
IPDHLSEPGKDFIRKCLQRDPSQRPTAMELLQHPFVQKAVSLEKSVLSEPLEHLAVISCRSSAK
MAAHTRNISSLGLEGQTIYQRRGAKFSSKHSDIRIRSNISCPVSPCGSPLLKSRSPQHSNGRMS
PSPISSPRTTSGTSTPLSGGNGAIPFNHLKQSTYSNEGFAIPSRSPDDLFASRPTDPDLGQFIR
VHQVSQGLQERVVSEADILSPQFGKRLGNVFDLRDKLSPSERFTHHAFVDHVKLNPSLDLTSG
SPHLGLKHGN

**INTERNATIONAL SEARCH REPORT**

International application No. **PCT/JP2024/035680**

**A. CLASSIFICATION OF SUBJECT MATTER**

***A01H 1/06***(2006.01)i; ***A01G 7/00***(2006.01)i; ***A01G 22/22***(2018.01)i; ***A01H 3/00***(2006.01)i; ***A01H 5/04***(2018.01)i; ***A01H 5/06***(2018.01)i; ***A01H 5/10***(2018.01)i; ***A01H 5/12***(2018.01)i; ***A01H 6/14***(2018.01)i; ***A01H 6/20***(2018.01)i; ***A01H 6/46***(2018.01)i; ***A01H 6/54***(2018.01)i; ***C07K 14/415***(2006.01)i; ***C12N 5/04***(2006.01)i; ***C12N 15/29***(2006.01)i; ***C12P 1/00***(2006.01)i; ***C12P 21/02***(2006.01)i

FI: A01H1/06; A01G7/00 601Z; A01G22/22 Z; A01H3/00; A01H5/04; A01H5/06; A01H5/10; A01H5/12; A01H6/14; A01H6/20; A01H6/46 ZNA; A01H6/54; C07K14/415; C12N5/04; C12N15/29; C12P1/00 Z; C12P21/02 C

According to International Patent Classification (IPC) or to both national classification and IPC

**B. FIELDS SEARCHED**

Minimum documentation searched (classification system followed by classification symbols)

A01H1/06; A01G7/00; A01G22/22; A01H3/00; A01H5/04; A01H5/06; A01H5/10; A01H5/12; A01H6/14; A01H6/20; A01H6/46; A01H6/54; C07K14/415; C12N5/04; C12N15/29; C12P1/00; C12P21/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X | SURIYASAK, Chetphilin et al., イネにおける登熟期の高温は次世代の玄米品質に及ぼす影響, 日本作物学会第249回講演会要旨集, 26 March 2020, p. 176, doi:10.14829/jcsproc.249.0_176, (Heat stress during grain filling induces transgenerational effects on rice (Oryza sativa L.) grain quality), non-official translation (Abstracts of The 249th Meeting of CSSJ) entire text | 1-8 |
| A | | 9, 10 |

☑ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2024** | **17 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/035680

C. DOCUMENTS CONSIDERED TO BE RELEVANT

| Category* | Citation of document, with indication, where appropriate, of the relevant passages | Relevant to claim No. |
|---|---|---|
| X | 坂井優光, 他, オオムギの高温登熟による子実内のDNAメチル化を介した種子発芽制御機構, 日本作物学会第253回講演会要旨集, 27 March 2022, p. 48, doi:10.14829/jcsproc.253.0_48, (SAKAI, Yuki et al., Heat Stress during Grain Filling in Barley (Hordeum vulgare L.) Regulates Seed Germination through Alterations of DNA Methylation), non-official translation (Abstracts of The 253rd Meeting of CSSJ) entire text | 1-8 |
| A | | 9, 10 |
| A | 澤田悠太, 他, イネにおける登熟期の低温ストレスによる次世代の低温登熟耐性, 日本作物学会第255回講演会要旨集, 29 March 2023, p. 105, doi:10.14829/jcsproc.255.0_105, (SAWADA, Yuta et al., Low temperature stress during grain filling induces transgenerational effects on rice (Oryza sativa L.) the low temperature tolerance during grain filling), non-official translation (Abstracts of The 255th Meeting of CSSJ) entire text | 1-10 |
| A | YANG, D. et al., Response of Photosynthesis to High Growth Temperature Was Not Related to Leaf Anatomy Plasticity in Rice (Oryza sativa L.), Frontiers in Plant Science, 07 February 2020, vol. 11, no. 26, pp. 1-9, doi:10.3389/fpls.2020.00026 entire text, all drawings | 1-10 |

**INTERNATIONAL SEARCH REPORT**

International application No.
**PCT/JP2024/035680**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9954471 A
- JP 2002512035 A
- WO 2011071050 A
- JP 5825574 B
- JP 2015181370 A
- WO 2023191038 A


### Non-patent literature cited in the description


- **KINOSHITA, T. et al.** Epigenetic Memory for Stress Response and Adaptation in Plants. *Plant Cell Physiol.*, 2014, vol. 55 (11), 1859-1863
- **SURIYASAK, C. et al.** Mechanism of delayed seed germination caused by high temperature during grain filling in rice (Oryza sativa L.). *Scientific Reports*, 2020, vol. 10, 17378
- Heat Priming Induces Trans-generational Tolerance to High Temperature Stress in Wheat. *Front. Plant Sci*, 2016, vol. 7
- **SAMAKOVLI et al.** *Molecular Plants*, 2020
- **ALTSCHUL et al.** *J. Mol. Biol.*, 1990, vol. 215, 403-410
- **SURIYASAK, C et al.** Mechanism of delayed seed germination caused by high temperature during grain filling in rice (Oryza sativa L.). *Sci. Rep*, 2020, vol. 10, 17378
- **ZHANG, X. et al.** Genome-wide-high-resolution mapping and functional analysis of DNA methylation in Arabidopsis. *Cell*, 2006, vol. 126, 1189-1201
- **GALINDO-GONZALEZ, L. et al.** LTR retrotransposons in plants: Engines of evolution. *Gene*, 2017, vol. 626, 14-25
- **EGASHIRA et al.** A rapid translocation of photo-assimilates from source organs maintains grain yield in cowpea subjected to drought stress during grain filling. *Biologia plantarum*, 2020, vol. 64, 529-534
- **SAMAKOVLI et al.** Molecular Plants YODA-HSP90 Module Regulates Phosphorylation-Dependent Inactivation of SPEECHLESS to Control Stomatal Development under Acute Heat Stress in Arabidopsis. *Molecular Plant*, 2020, vol. 13, 612-633